(19) Europäisches Patentamt
European Patent Office
Office européen des brevets

(11) **EP 0 995 451 B1**

(12) **EUROPÄISCHE PATENTSCHRIFT**

(45) Veröffentlichungstag und Bekanntmachung des Hinweises auf die Patenterteilung:
**07.12.2005  Patentblatt 2005/49**

(51) Int Cl.$^7$: **A61M 1/16**, A61M 1/36

(21) Anmeldenummer: **99120600.4**

(22) Anmeldetag: **16.10.1999**

(54) **Vorrichtung zur Überwachung eines Gefässzuganges**

Device for monitoring a blood vessel access

Dispositif de surveillance d'un accès à un vaisseau sanguin

(84) Benannte Vertragsstaaten:
**DE ES FR GB IT**

(30) Priorität: **20.10.1998  DE 19848235**

(43) Veröffentlichungstag der Anmeldung:
**26.04.2000  Patentblatt 2000/17**

(60) Teilanmeldung:
**05013560.7 / 1 584 339**

(73) Patentinhaber: **Fresenius Medical Care Deutschland GmbH**
**61352 Bad Homburg v.d.H. (DE)**

(72) Erfinder: **Kleinekofort, Wolfgang, Dr.**
**65779 Kelkheim (DE)**

(74) Vertreter: **Luderschmidt, Schüler & Partner GbR Patentanwälte,**
**John-F.-Kennedy-Strasse 4**
**65189 Wiesbaden (DE)**

(56) Entgegenhaltungen:
**EP-A- 0 611 228**          **WO-A-97/10013**
**DE-A- 4 024 434**          **DE-C- 19 734 002**

**Beschreibung**

Die Erfindung betrifft eine Vorrichtung zur extrakorporalen Blutbehandlung, insbesondere zur einer chronischen Blutreinigungstherapie wie Hämodialyse, Hämofiltration und Hämodiafiltration mit einer Einrichtung zur Überwachung des Gefäßzuganges.

Bei den bekannten Methoden der chronischen Blutreinigungstherapie wie Hämodialyse, Hämofiltration und Hämodiafiltration wird Blut des Patienten über einen extrakorporalen Kreislauf geleitet. Als Zugang zum Gefäßsystem des Patienten werden arteriovenöse Fisteln, Gefäßimplantate oder auch verschiedene Katheter verwendet. Typische Flüsse innerhalb des Gefäßzugangs liegen im Bereich von 1100 ml/min. Die Konnektion des Patienten mit dem extrakorporalen Kreislauf erfolgt in der Regel über Dialysekanülen, mit denen die Fistel bzw. das Gefäßimplantat punktiert wird.

Falls sich während der Behandlung der Anschluß zwischen extrakorporalem Kreislauf und Gefäßsystem löst bzw. ein Blutleck im extrakorporalen Kreislauf auftritt, kann ein Verbluten des Patienten nur verhindert werden, wenn innerhalb weniger Minuten der extrakorporale Blutfluß gestoppt wird. Daher sind extrakorporale Blutkreisläufe im allgemeinen mit Schutzsystemen ausgestattet, die permanent den arteriellen und venösen Druck ($P_{art.}$ bzw. $P_{ven.}$) innerhalb des Systems sowie den Eintritt von Luft in den extrakorporalen Kreislauf überwachen.

Im Alarmfall wird die Blutbehandlung gestoppt, die venöse Klemme geschlossen sowie ein akustisches und optisches Warnsignal ausgelöst. Das auf der Druckmessung basierende Schutzsystem spricht an, wenn sich der arterielle oder venöse Druck im extrakorporalen Kreislauf um mehr als ± 60 Torr ändert. Hierbei sind die Alarmgrenzen so gewählt, daß eine Lageänderung des Patienten keinen Alarm auslöst.

Falls sich der Anschluß zwischen Patient und Maschine an der arteriellen Verbindung löst, d. h. an derjenigen Kanüle, die den Blutfluß vom Patienten zum extrakorporalen Kreislauf herstellt, spricht das druckbasierende maschinenseitige Schutzsystem schnell an, wie im folgenden begründet wird. Die Dialysekanüle stellt den höchsten Flußwiderstand im extrakorporalen System dar. Wenn Luft über die Kanüle in das arterielle Unterdrucksystem des extrakorporalen Kreislaufs eingesogen wird, sinkt der Flußwiderstand der Kanüle proportional zum Dichteunterschied zwischen Blut und Luft um den Faktor $10^3$. Somit bricht der arterielle Unterdruck im extrakorporalen Kreislauf schlagartig zusammen.

Im Fall, daß sich die venöse Kanüle aus dem Gefäßzugang löst, ist das Ansprechen des druckbasierenden Schutzsystems jedoch nicht immer gewährleistet. Auf der venösen Seite wird das gereinigte Blut dem Patienten mit Überdruck zugeführt, wobei der venöse Überdruck proportional zur Fördermenge der Blutpumpe ist. Ein Eindringen von Luft durch die Kanüle in den extrakorporalen Kreislauf -wie es auf der arteriellen Unterdruckseite der Fall wäre- ist somit ausgeschlossen. Daher ändert sich der Flußwiderstand der venösen Kanüle nicht und der venöse maschinenseitige Druck sinkt lediglich um den Betrag des Drucks im Gefäßzugang des Patienten. Somit ist die venöse Druckänderung im extrakorporalen Kreislauf in der Regel zu klein, um ein Ansprechen des druckbasierenden Schutzsystems auszulösen. Nur im Fall, daß die venöse Kanüle nach dem Herausrutschen aus dem Gefäßzugang deutlich unterhalb der Fistel liegt, bewirkt die zusätzliche hydrostatische Druckdifferenz zwischen venösem Drucksensor und Kanüle einen Maschinenalarm.

Auch im Falle eines Blutlecks im venösen Schlauchsystem kann es vorkommen, daß der resultierende venöse Druckabfall nicht ausreicht, um ein Auslösen des vorhandenen druckbasierenden Schutzsystems zu gewährleisten.

Neben dem obigen Verfahren, bei dem der Druck im arteriellen Zweig des extrakorporalen Kreislaufs überwacht wird, um ein Herausrutschen der arteriellen Kanüle zu erkennen und unabhängig von der Drucküberwachung im arteriellen Zweig der Druck im venösen Zweig des extrakorporalen Kreislaufs überwacht wird, um ein Herausrutschen der venösen Kanüle zu erkennen, sind Überwachungssysteme bekannt, die sich im extrakorporalen Kreislauf fortpflanzende Druckpulse überwachen.

Die WO 97/10013 beschreibt eine Dialysevorrichtung mit einem derartigen Überwachungssystem. das die in der arteriellen Blutleitung durch die Blutpumpe erzeugten Druckpulse in der venösen Blutleitung überwacht

Die EP 0 611 228 A2 beschreibt eine Blutbehandlungsvorrichtung, die über Drucksensoren im venösen und arteriellen Zweig des extrakorporalen Blutkreislaufs verfügt. Die digitalisierten Ausgangssignale der Drucksensoren werden in einem Monitor-Prozessor verarbeitet, der das gesamte System überwacht. Beim Auftreten eines Fehlalarms gibt der Prozessor Alarm. Zu einer der Alarmbedingungen gehört der korrekte Anschluss der venösen und arteriellen Blutleitung. Hierzu wird vorgeschlagen, den Messwert für den Druck der jeweiligen Leitung mit einem vorgegebenen Referenzwert zu vergleichen.

Der Erfindung liegt die Aufgabe zugrunde eine Vorrichtung zur extrakorporalen Blutbehandlung mit einer Einrichtung zur Überwachung eines Gefäßzuganges zu schaffen, die sowohl ein Herausrutschen der venösen Kanüle aus dem Gefäßzugang als auch ein Blutleck im venösen Zweig des extrakorporalen Kreislaufs mit hoher Zuverlässigkeit erkennt und nur einen geringen apparativen Aufwand erfordert. Die Lösung dieser Aufgabe erfolgt erfindungsgemäß mit den Merkmalen des Patentanspruchs 1.

Die Einrichtung für die erfindungsgemäße Blutbehandlungsvorrichtung kann als maschinenintegriertes Schutzsystem ausgelegt sein. Hierbei wird von Meßfühlern Gebrauch gemacht, die bereits in den bekannten Blutbehandlungsvorrichtungen vorhanden sind. Somit beschränkt sich die maschinenseitige Änderung zur Implementierung des Schutzsystems lediglich auf eine Modifikation der Maschinensteuerung.

Die erfindungsgemäße Vorrichtung beruht darauf, daß sowohl der Druck im arteriellen Zweig als auch der Druck im venösen Zweig des extrakorporalen Kreislaufs überwacht wird, um ein Herausrutschen der venösen Kanüle aus dem Gefäßzugang oder ein Blutleck im venösen Zweig des extrakorporalen Kreislaufs zu erkennen. Aus dem Druck im arteriellen und venösen Zweig des extrakorporalen Kreislaufs werden für den Zustand des Gefäßzugangs charakteristische Werte berechnet, die dann zur Erkennung eines fehlerhaften Gefäßzuganges ausgewertet werden.

Mit der erfindungsgemäßen Vorrichtung kann nicht nur ein Herausrutschen der venösen Kanüle und ein Blutleck im venösen Zweig des extrakorporalen Kreislaufs sicher erkannt werden, sondern auch ein Herausrutschen der arteriellen Kanüle und ein Blutleck im arteriellen Zweig des extrakorporalen Kreislaufs.

Das Verfahren auf dem die erfindungsgemäße Vorrichtung beruht, kann auch mit anderen Verfahren zur Erkennung eines fehlerhaften Gefäßzuganges kombiniert werden. Damit wird die Sicherheit des Überwachungssystems noch weiter erhöht.

Für den Fall, daß der Gefäßzugang fehlerhaft ist, wird vorzugsweise ein akustischer und/oder optischer Alarm gegeben. Darüber hinaus kann der Blutfluß im extrakorporalen Kreislauf unterbrochen werden, um einen Blutverlust zu vermeiden. Eine Unterbrechung des Blutflusses ist bei den bekannten Vorrichtungen zur extrakorporalen Blutbehandlung dadurch möglich, daß die im extrakorporalen Kreislauf angeordnete Blutpumpe angehalten und/oder ein im extrakorporalen Kreislauf angeordnetes Sicherheitsventil, z. B. eine Schlauchklemme, geschlossen wird.

Eine Überwachung des fehlerhaften Gefäßzuganges mit dem Verfahren auf dem die erfindungsgemäße Vorrichtung beruht kann nicht nur in Vorrichtungen zur Hämodialyse, Hämofiltration und Hämodiafiltration erfolgen, sondern auch in den bekannten Zellseparatoren, in denen das Blut eines Spenders in einem extrakorporalen Kreislauf einer Zentrifugation unterworfen und dabei in seine Bestandteile getrennt wird.

Im folgenden wird das Verfahren zur Überwachung eines Gefäßzuganges auf dem die erfindungsgemäße Vorrichtung beruht, sowie die erfindungsgemäße Vorrichtung zur extrakorporalen Blutbehandlung mit einer Einrichtung zur Überwachung des Gefäßzuganges unter Bezugnahme auf die Zeichnungen anband eines Ausführungsbeispiels näher erläutert.

Es zeigen:

Fig. 1 eine Tabelle, aus der die Einflüsse ersichtlich sind, die zu einer Änderung des Druckes im arteriellen und venösen Zweig des extrakorporalen Kreislaufs beitragen,

Fig. 2 ein Ausführungsbeispiel einer Vorrichtung zur extrakorporalen Blutbehandlung mit einer Einrichtung zur Überwachung des Gefäßzuganges in vereinfachter schematischer Darstellung,

Fig. 3 einen Flußlaufplan der Überwachungseinrichtung,

Fig. 4 den arteriellen und venösen Druck in Abhängigkeit vom Blutfluß im extrakorporalen Kreislauf,

Fig. 5 die Änderung des arteriellen und venösen Drucks im extrakorporalen Kreislauf bei einer Lageänderung des Patienten,

Fig. 6 die Druckverhältnisse im extrakorporalen Kreislauf beim Herausrutschen der venösen Kanüle aus dem Gefäßzugang und

Fig. 7 die Druckverhältnisse bei einem Blutleck im venösen Zweig des extrakorporalen Kreislaufs.

Während der extrakorporalen Blutbehandlung werden der arterielle und venöse Druck $P_{ven.} + P_{art.}$ im extrakorporalen Kreislauf mit einer Frequenz f gemessen und die Summe $P_S$ aus venösem und arteriellem Druck gebildet (Gleichung 1).

$$P_S = P_{ven.} + P_{art.} \qquad \text{(Gleichung 1)}$$

$P_{art.}$: Meßwert des arteriellen Drucks im extrakorporalen Kreislauf.
$P_{ven.}$: Meßwert des venösen Drucks im extrakorporalen Kreislauf.

Zusätzlich wird die Differenz $\Delta P$ des venösen und arteriellen Drucks berechnet:

$$\Delta P = P_{ven.} - P_{art.} \qquad \text{(Gleichung 2)}$$

Die Änderung der Summe aus arteriellem und venösem Druck ist gegeben durch:

$$d(P_S) = P_{SN} - P_{SN-1} \qquad \text{(Gleichung 3)}$$

$P_{SN}$: Aktueller Meßwert der Drucksumme.
$P_{SN-1}$: Vorheriger Meßwert der Drucksumme.

Die Änderung der Druckdifferenz beträgt:

$$d(\Delta P) = \Delta P_N - \Delta P_{N-1} \quad \text{(Gleichung 4)}$$

$\Delta P_N$: Aktueller Meßwert der Druckdifferenz.
$\Delta P_{N-1}$: Vorheriger Meßwert der Druckdifferenz.

Auf einen fehlerhaften Gefäßzugang wird dann geschlossen, wenn für N aufeinanderfolgende Messungen die folgenden Bedingungen erfüllt sind:

a) $\Sigma N \, d(P_s)$ ist negativ und kleiner als ein Schwellenwert $M_1$ (Gleichung 5)
b) $\Sigma N \, d(\Delta P)$ ist negativ und kleiner als ein Schwellenwert $M_2$ (Gleichung 6)

In diesem Fall ist entweder die venöse Kanüle herausgerutscht oder es liegt ein Blutleck im venösen Zweig des extrakorporalen Kreislaufs vor.

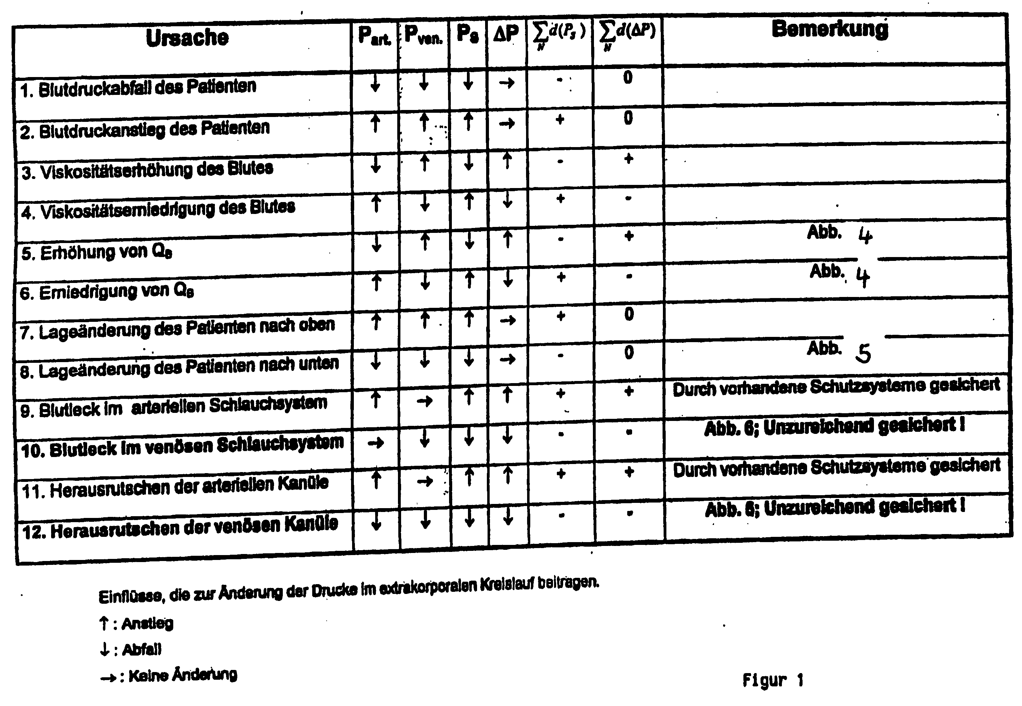

Aus der Tabelle (Figur 1) geht hervor, welche Einflüsse zur Änderung des venösen und arteriellen Drucks im extrakorporalen Kreislauf beitragen. Aus der Tabelle ist ersichtlich, daß neben dem Herausrutschen der venösen Kanüle und einem Blutleck im venösen Zweig auch ein Herausrutschen der arteriellen Kanüle und ein Blutleck im arteriellen Zweig des extrakorporalen Kreislaufs erkannt werden können.

Auf ein Herausrutschen der arteriellen Kanüle oder ein Blutleck im arteriellen Zweig des extrakorporalen Kreislaufs kann dann geschlossen werden, wenn die folgenden Bedingungen erfüllt sind:

a) $\Sigma N \, d(P_s)$ ist positiv und größer als ein Schwellenwert $M_3$ (Gleichung 7)
b) $\Sigma N \, d(\Delta P)$ ist positiv und größer als ein Schwellenwert $M_4$ (Gleichung 8)

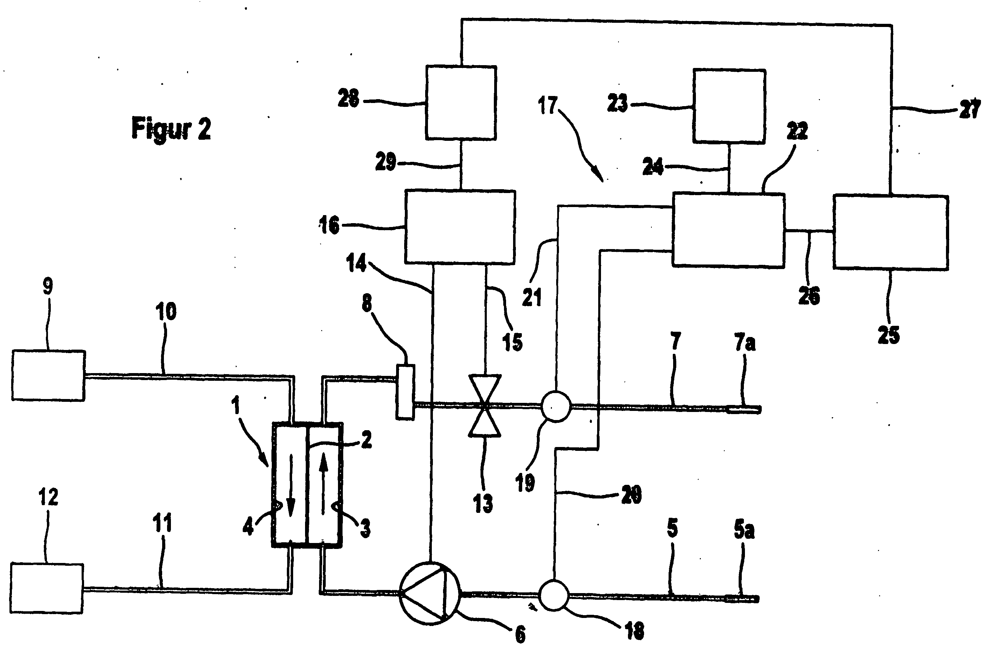

Figur 2 zeigt eine vereinfachte schematische Darstellung einer Dialysevorrichtung mit einer Einrichtung zur Überwachung des Gefäßzuganges.

Die Dialysevorrichtung weist als Blutbehandlungseinrichtung einen Dialysator 1 auf, der durch eine semipermable Membran 2 in eine Blutkammer 3 und eine Dialysierflüssigkeitshmmer 4 unterteilt ist. An dem Einlaß der Blutkammer ist eine arterielle Blutleitung 5 angeschlossen, in die eine peristaltische Blutpumpe 6 geschaltet ist. Stromab der Blutkammer 3 führt eine venöse Blutleitung 7 von dem Auslaß der Blutkammer zu dem Patienten. In die venöse Blutleitung 7 ist eine Tropfkammer 8 geschaltet. An den Enden der arteriellen und venösen Blutleitung 5, 7 sind Kanülen 5a, 7a angeschlossen, die in den Patienten gestochen werden. Die arterielle und venöse Blutleitung sind Bestandteil eines als Disposible ausgebildeten Schlauchleitungssystems.

In einer Dialysierflüssigkeitsquelle 9 wird frische Dialysierflüssigkeit bereitgestellt. Von der Dialysierflünigkeitsquelle 9 führt eine Dialysierflüssigkeitzuführleitung 10 zu dem Eingang der Dialysierflüssigkeitskammer 4 des Dialysators 1, während eine Dialisierflüssigkeitabführleitung 11 von dem Ausgang der Dialysierflüssigkeitskammer zu einem Abfluß 12 führt.

Die Dialysevorrichtung kann noch über weitere Komponenten, z. B. eine Bilanziereinrichtung und eine Ultrafiltrationseinrichtung etc., verfügen, die der besseren Übersichtlichkeit halber nicht dargestellt sind.

Zur Unterbrechung des Blutflusses ist an der venösen Blutleitung 7 stromab der Tropfkammer 8 eine Absperrklemme 13 vorgesehen, die elektromagnetisch betätigt wird. Die arterielle Blutpumpe 6 und die venöse Absperrklemme 13 werden über Steuerleitungen 14, 15 von einer Steuereinheit 16 angesteuert.

Die Einrichtung 17 zur Überwachung des Gefäßzuganges weist einen den Druck in der arteriellen Blutleitung 5 überwachenden arteriellen Drucksensor 18 und einen den Druck in der venösen Blutleitung 7 überwachenden venösen Drucksensor 19 auf. Die Meßwerte der Drucksensoren 18, 19 werden über Datenleitungen 20, 21 an eine Recheneinheit 22 übertragen, die aus den Meßwerten für den Zustand des Gefäßzugangs charakteristische Werte berechnet. In einer Speichereinheit 23, die über eine Datenleitung 24 mit der Recheneinheit 22 verbunden ist, werden die bei der Berechnung anfallenden Zwischenergebnisse abgelegt. Zur Auswertung der für den Gefäßzugang charakteristischen Werte weist die Überwachungseinheit eine Auswerteinheit 25 auf, die über eine Datenleitung 26 mit der Recheneinheit verbunden ist. Die Auswerteinheit 25 ist über eine Steuerleitung 27 an eine Alarmeinheit 28 angeschlossen, die über eine Steuerleitung 29 mit der Steuereinheit 16 verbunden ist.

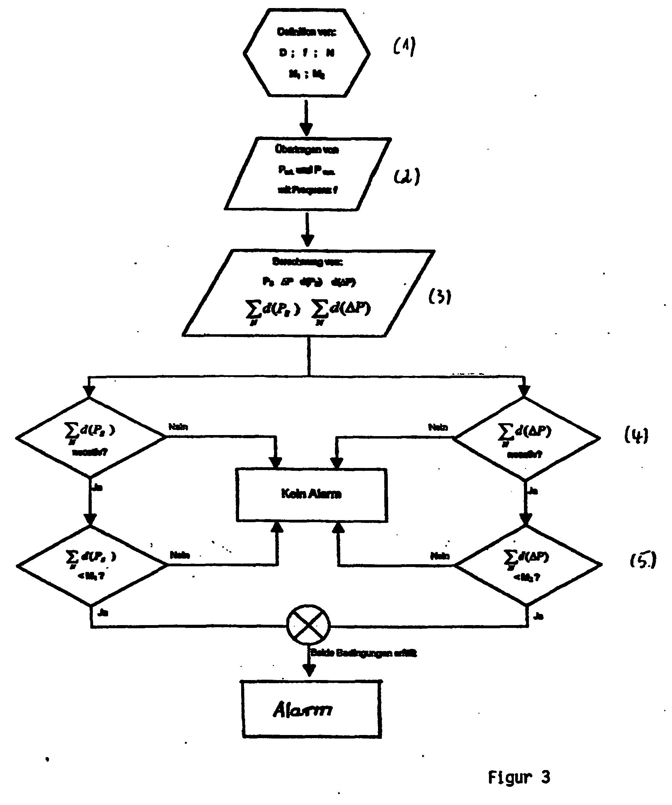

Nachfolgend wird die Funktionsweise der Überwachungseinrichtung 17 unter Bezugnahme auf Figur 3 im einzelnen beschrieben.

Zunächst werden die für die Auswertung heranzuziehende Anzahl N aufeinanderfolgender Meßwerte, die Meßfrequenz f sowie die Schwellwerte $M_1$ und $M_2$ festgelegt. Diese Werte können in der Speichereinheit 23 abgelegt sein oder auch vom Benutzer vorgegeben werden (Schritt 1).

Durch die Auswertung von N aufeinanderfolgenden Meßwerten wird die Störanfälligkeit des Schutzsystems, z. B. bei kurzzeitigen artifiziellen Druckschwankungen reduziert. Der Wert für N ist ganzzahlig und abhängig von der Frequenz f, mit welcher die Druckwerte ermittelt werden. Er kann der jeweiligen Dämpfung D der Drucksensoren angepaßt werden. Für N gilt fogende Randbedingung:

$$N > f \cdot D \quad \text{(Gleichung 9)}$$

Bei einer typischen Meßfrequenz f von z. B. 1/3 Hz und einer Dämpfung von ca. 18 s sollte N folglich > 6 sein. Hierdurch ist gewährleistet, daß der Druckabfall vollständig erkannt wird.

Die negativen Schwellenwerte $M_1$ und $M_2$

kennzeichnen die Empfindlichkeit des Schutzsystems. Generell gilt: Je kleiner $M_1$ und $M_2$, desto höher liegt die Schwelle, bei welcher ein Maschinenalarm ausgelöst wird. Um ein Ansprechen des Schutzsystems zu gewährleisten, müssen die Werte für $M_1$ und $M_2$ jedoch größer als der negative Wert des venösen Drucks im Gefäßzugang sein.

Der arterielle Fisteldruck sinkt nach dem Herausrutschen der venösen Kanüle aus dem Gefäßzugang durch die Blutung an der venösen Punktionsstelle geringfügig ab. Dieser Effekt kann durch die geeignete Wahl von $M_2$ berücksichtigt werden, wobei die Bedingung $M_1 < M_2$ gelten muß. Geeignete Werte sind z. B. $M_1$=-15 Torr und $M_2$=-10 Torr.

Die Ansprechzeit des Schutzsystems $\tau$ ist gegeben durch:

$$\tau = \frac{N}{f} \qquad \text{(Gleichung 10)}$$

Während der Dialysebehandlung werden mittels der Druckmeßsensoren 18, 19 der arterielle und venöse Druck $P_{art.}$, $P_{ven.}$ mit der Meßfrequenz f in N aufeinanderfolgenden Messungen erfaßt (Schritt 2).

Die Recheneinheit 22 berechnet nach jeder Messung gemäß Gleichung 1 die Summe $P_S$ des venösen und arteriellen Drucks und gemäß Gleichung 2 die Differenz $\Delta P$ des venösen und arteriellen Drucks. Diese Werte werden in der Speichereinheit 23 abgelegt. Nach Gleichung 3 berechnet die Recheneinheit 22 die Differenz $d(P_S)$ zwischen der Summe $P_{SN}$ des venösen und arteriellen Drucks einer nachfolgenden Messung und der Summe $P_{SN-1}$ des venösen und arteriellen Drucks einer vorhergehenden Messung. Die Differenz $d(\Delta P)$ zwischen der Differenz $d(\Delta P)$ des arteriellen und venösen Drucks einer vorhergehenden Messung $\Delta P_N$ und der Differenz $\Delta P_{N-1}$ des arteriellen und venösen Drucks einer nachfolgenden Messung berechnet die Recheneinheit 22 nach Gleichung 4. Auch diese Meßwerte werden in der Speichereinheit 23 abgelegt. Die Summenänderungen $d(P_s)$ der N aufeinanderfolgenden Messungen werden dann zur Berechnung eines ersten den Zustand des Gefäßzuganges charakteristischen Wertes $W_1$ in der Recheneinheit 22 nach Gleichung 5 addiert. Zur Berechnung eines den Gefäßzustand charakteristischen zweiten Wertes $W_2$ werden in der Recheneinheit 22 die Differenzänderungen $d(\Delta P)$, nach Gleichung 6 addiert (Schritt 3).

Die Auswerteinheit 25 überprüft das Vorzeichen der beiden charakteristischen Werte $W_1$ und $W_2$. Sind der erste oder der zweite charakteristische Wert $W_1$, $W_2$ negativ, so erfolgt kein Alarm (Schritt 4). Wenn jedoch sowohl der erste als auch der zweite charakteristische Wert $W_1$, $W_2$ negativ sind, werden die charakteristischen Werte in der Auswerteinheit 25 mit dem ersten und zweiten Schwellwert M1, M2 verglichen (Schritt 5).

Für den Fall, daß sowohl $W_1$ kleiner als $M_1$ als auch $W_2$ kleiner $M_2$, gibt die Auswerteinheit 25 ein Alarmsignal an die Alarmeinheit 28 ab. Die Alarmeinheit 28 erzeugt einen akustischen und oder optischen Alarm und steuert die Steuereinheit 16 an, die wiederum die Blutpumpe 6 anhält und das Absperrorgan 13 schließt. Damit ist sichergestellt, daß in dem Fall, daß die venöse Kanüle 7a herausgerutscht ist oder ein Leck in der venösen Blutleitung 7 vorliegt der Patient nicht gefährdet ist.

In analoger Weise kann nach Gleichungen 7 und 8 auf einen fehlerhaften Gefäßzustand aber auch dann geschlossen werden, wenn sowohl der erste charakteristische Wert $W_1$ als auch der zweite charakteristische Wert $W_2$ positiv sind und der erste charakteristische Wert $W_1$ größer als ein vorgegebener Schwellwert $M_3$ und der zweite charakteristische Wert $W_2$ größer als ein vorgegebener Schwellwert $M_4$ ist. In diesem Fall ist die arterielle Kanüle herausgerutscht oder es liegt ein Blutleck im arteriellen Zweig des extrakorporalen Kreislaufs vor.

Die Recheneinheit 22 mit der Speichereinheit 23 und die Auswerteinheit 25 können Bestandteil des Mikrocomputers sein, der in den bekannten Dialysevorrichtungen ohnehin vorhanden ist.

Sofern die Dialysevorrichtung über eine Ultrafiltrationseinrichtung verfügt, wird die Überwachungseinrichtung innerhalb der ersten ein bis zwei Minuten nach dem Ein- bzw. Ausschalten der Ultrafiltrationseinrichtung deaktiviert, um einen Fehlalarm zu vermeiden.

Nachfolgend werden die charakteristischen Grundlagen erläutert, auf denen das Verfahren zur Überwachung des Gefäßzugangees beruht, auf dem die erfindungsgemäße Vorrichtung beruht.

Die im extrakorporalen Kreislauf gemessenen Drucke $P_{art.}$ und $P_{ven.}$ setzen sich aus dem dynamischen Druck im Extrakorporalsystem, der durch den Fluß der Blutpumpe erzeugt wird, und dem dynamischen Druck im Gefäßzugang des Patienten zusammen.

Hierbei ist der dynamische Druck im Extrakorporalsystem eine Funktion des extrakorporalen Blutflusses sowie der Summe der Flußwiderstände im extrakorporalen Kreislauf. Da sich arterieller und venöser Flußwiderstand aufgrund der unterschiedlichen Geometrie der durchströmten Komponenten unterscheiden, ist die Drucksumme $P_s$ ebenfalls eine Funktion des Blutflusses.

In der Regel wird bei den bekannten Blutreinigungsverfahren die Förderrate der Blutpumpe $Q_B$ auf einen festen Wert eingestellt. Somit ist die Summe der Flußwiderstände im extrakorporalen Kreislauf bei konstanter Viskosität des Blutes ebenfalls konstant. Eine Erhöhung der Viskosität, z. B. durch Ultrafiltration, führt langfristig zur Erniedrigung des arteriellen Unterdrucks und zur Erhöhung des venösen Überdrucks. Folglich ist das Druckverhalten bei stetiger Viskositätserhöhung identisch mit einer geringen Erhöhung des Blutflusses.

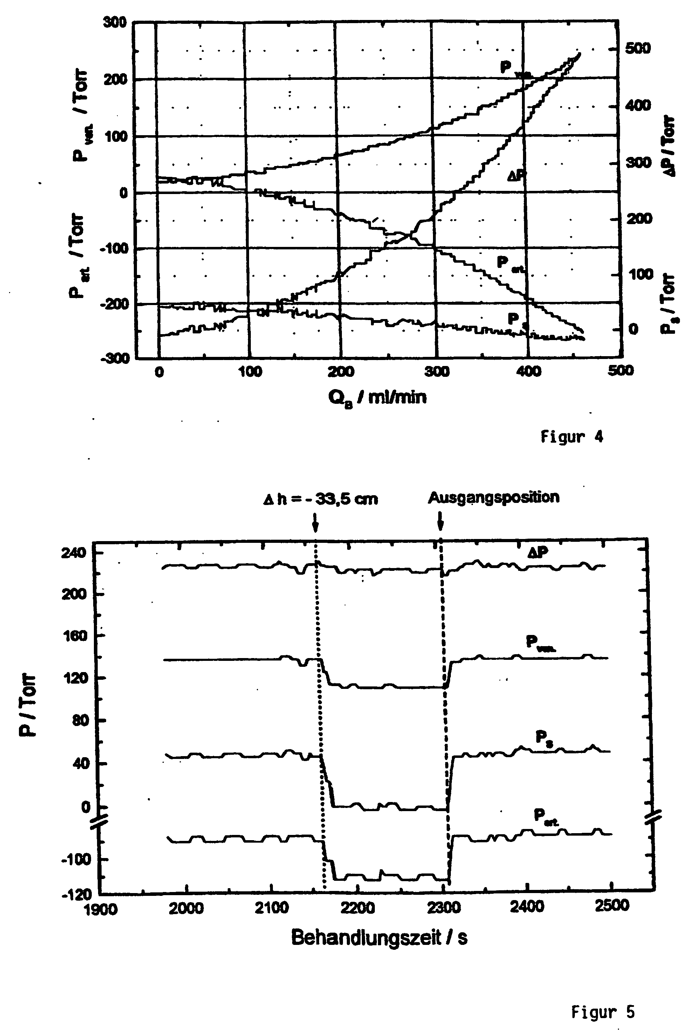

Figur 4 zeigt die extrakorporalen Drucke in Abhängigkeit vom Blutfluß $Q_B$ bei konstanter Viskosität (Fistelfluß $Q_F$ = 1255 ml/min, arterieller Fisteldruck $P_{Fart.}$ = 27 Torr, venöser Fisteldruck $P_{Fven.}$ = 17 Torr).

Die obige und alle folgenden Messungen wurden während einer simulierten Dialysebehandlung durchgeführt. Als Gefäßzugang wurde ein Schlauchsegment mit einem Innendurchmesser von 8 mm verwendet, welches mit einer Zahnradpumpe verbunden war. Zur Messung des Flusses innerhalb des Gefäßzugangs wurde ein Doppler-Flußmesser verwendet. Die arteriellen und venösen Drucke innerhalb des Gefäßzugangs ($P_{Fistel}$) wurden mit zwei Druckmanometern kontrolliert und konnten über Schlauchklemmen eingestellt werden. Bei allen Messungen wurde Wasser verwendet (T=37°C). Zur Gefäßpunktion wurden Kanülen verwendet, die über zwei konventionelle Schlauchsysteme mit dem Dialysegerät verbunden waren.

Der dynamische Druck im Gefäßzugang des Patienten, im folgenden Fisteldruck genannt, ist ebenfalls eine Funktion der Blutviskosität, des systemischen Blutdrucks sowie der systemischen vaskulären Flußwiderstände. Der Fisteldruck ist somit ein patientenspezifischer Parameter und hängt zusätzlich ab von der Art des Gefäßzugangs, der Viskosität des Blutes sowie vom Gefäßsystem, das den Gefäßzugang mit Blut versorgt. Analog zum dynamischen Druck im Extrakorporalsystem führt eine Änderung des Fisteldrucks, z. B. durch Blutdruckschwankung, Viskositätserhöhung oder Lageänderung des Patienten zur Änderung sowohl des arteriellen als auch des venösen Druckwertes.

Figur 5 zeigt das Verhalten der extrakorporalen Drucke bei konstantem effektiven Blutfluß $Q_B$, wenn sich die Lage des Patienten relativ zu den extrakorporalen Drucksensoren um Δh=-33,5 cm ändert ($Q_B$= 300 ml/min, $Q_F$=1252 ml/min; Druck im Gefäßzugang: $P_{F\,art.}$ = 27 Torr, $P_{F\,ven.}$ = 17 Torr). Dies ist z. B. der Fall, wenn sich der Patient hinlegt bzw. seine Liege herunterfährt. Hierbei reduzieren sich die arteriellen und venösen Druckwerte um den Betrag der hydrostatischen Druckdifferenz (ca. 0,78 Torr pro cm Höhendifferenz zwischen Drucksensor und Fistel). Da sich bei einer Lageänderung des Patienten der arterielle und venöse Druck im extrakorporalen Kreislauf um den gleichen Wert ändert, bleibt die Druckdifferenz ΔP konstant. Hingegen sinkt die Summe der Drucke $P_S$ um den doppelten Betrag der hydrostatischen Druckdifferenz.

Dieses Verhalten zeigt sich (allerdings in abgeschwächter Form) auch bei einem Blutdruckabfall während der Behandlung. In diesem Falle sinken ebenfalls der arterielle und venöse Druck im extrakorporalen Kreislauf.

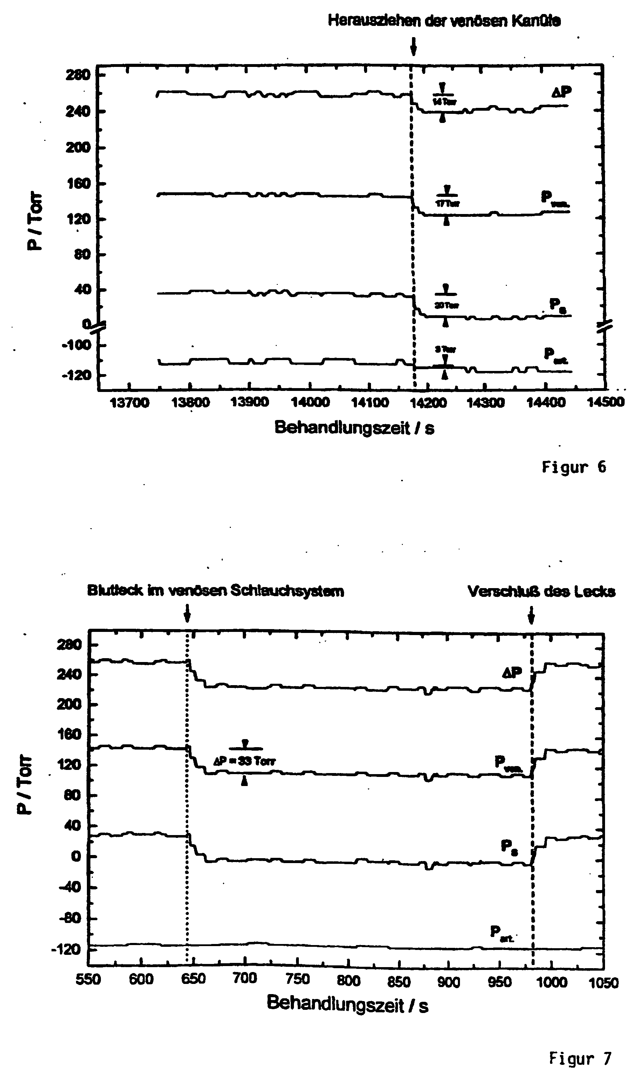

Figur 6 zeigt die Druckverhältnisse beim Herausrutschen der venösen Dialysekanüle aus der Fistel des Patienten ($Q_B$=300 ml/min, $Q_F$=1254 ml/min, $P_{F\,art.}$=27 Torr, $P_{F\,ven.}$=17 Torr). Der Druck am venösen Sensor des extrakorporalen Kreislaufs ($P_{ven.}$) verringert sich innerhalb von ca 15-20 Sekunden um den Betrag des venösen Fisteldrucks. Die Verzögerungszeit ist durch die elektronische Dämpfung der extrakorporalen Drucksignale bedingt. Der arterielle Druckwert ($P_{art.}$) reduziert sich innerhalb der ersten Minuten nur geringfügig, wie im folgenden begründet wird.

Der Blutverlust, welcher durch die offene venöse Punktionsstelle bedingt ist und zum Absinken des Fisteldrucks führt, kann über den Energieerhaltungssatz grob abgeschätzt werden:

$$P \cdot V = \frac{1}{2} M \cdot v^2 \qquad (Gleichung\ 11)$$

mit folgenden Abkürzungen:

P: Druck
V: Volumen
M: Masse
v: Geschwindigkeit

Mit V=M/ρ folgt für die Geschwindigkeit des Blutstrahls aus der offenen Punktionsstelle:

$$v = \sqrt{\frac{2P}{\rho}} \qquad (Gleichung\ 12)$$

ρ: Dichte

Bei einem venösen Fisteldruck von 17 Torr (=22,6 mbar) und einer Blutdichte von 1,0506 g/cm$^3$ (37°C) beträgt die Strahlgeschwindigkeit ca. 2,1 m/s. Unter der Annahme einer gleichbleibenden Öffnung der Punktionsstelle ist der Volumenstrom bei zylindrischem Strahlprofil gegeben durch:

$$Q = \pi \cdot r^2 \cdot v \qquad (Gleichung\ 13)$$

Bei einem typischen Kanülendurchmesser von 1,6 ergibt sich ein Wert von ca. 250 ml/min. (= 1/5 des Fistelflusses). In vivo schwillt die offene Punktionsstelle an, wodurch der effektive Lochdurchmesser kleiner als der Kanülendurchmesser sein wird. Somit ist der angegebene Wert als Obergrenze anzusehen.

Da der Druck und Fluß proportional sind, reduziert sich der venöse Fisteldruck folglich um 17/5 = 3,4 Torr (Gesetz von Hagen-Poiseuille). Durch den verminderten venösen Fisteldruck sinkt der arterielle extrakorporale Druck um den gleichen Wert. Da die arterielle Druckänderung jedoch immer kleiner als die venöse Druckänderung ist, verringern sich sowohl $P_S$ als auch ΔP.

Figur 7 zeigt die Druckverhältnisse bei einem Blutleck im venösen Schlauchsystem. Hierzu wurde der venöse Schlauch unterhalb der Tropfkammer mit einer Injektionskanüle punktiert ($Q_B$=300 ml/min, $Q_F$=1250ml/min, $P_{F\,art.}$ = 27 Torr, $P_{F\,ven.}$ = 17 Torr, Leckrate 50 ml/min). Der Druck am venösen Sensor verringert sich bei einer Leckrate von 50 ml/min innerhalb von ca. 15-20 s um rund 33 Torr. Der arterielle Druck hingegen ändert sich nicht merklich. Analog zu Figur 6 sinken sowohl ∆P als auch $P_S$, folglich sind die Summenänderungen nach Gleichung 6 und 7 in beiden Fällen negativ.

**Patentansprüche**

1. Vorrichtung zur extrakorporalen Blutbehandlung mit
einer arteriellen Blutleitung (5) eines extrakorporalen Blutkreislaufs, die an einem Ende mit dem Einlaß einer Blutbehandlungseinrichtung (1) verbunden ist und an dem anderen Ende mit einem arteriellen Anschluß (5a) für einen Gefäßzugang versehen ist,
einer venösen Blutleitung (7) des extrakorporalen Blutkreislaufs, die an einem Ende mit dem Auslaß der Blutbehandlungseinrichtung (1) verbunden ist und an dem anderen Ende mit einem venösen Anschluß (7a) für den Gefäßzugang versehen ist, und einer Einrichtung (17) zur Überwachung des Gefäßzuganges mit einem den Druck $P_{art.}$ in der arteriellen Blutleitung überwachenden arteriellen Drucksensor (18) und einen den Druck $P_{ven.}$ in der venösen Blutleitung überwachenden venösen Drucksensor (19),
**dadurch gekennzeichnet,**
**daß** die Einrichtung zur Überwachung des Gefäßzuganges aufweist:

eine Recheneinheit (22) mit einem Mittel zum Berechnen der Summe und der Differenz des in aufeinanderfolgenden Messungen erfaßten venösen und arteriellen Drucks im extrakorporalen Kreislauf und Mitteln zum Berechnen von für den Zustand des Gefäßzugangs charakteristischen Werten $W_1$, $W_2$ aus den ermittelten Summen und Differenzen der erfassten Druckwerte im arteriellen und venösen Zweig des extrakorporalen Kreislaufs und
eine Auswerteinheit (25) zur Auswertung der charakteristischen Werte, um einen fehlerhaften Gefäßzugang zu erkennen.

2. Vorrichtung nach Anspruch 1, **dadurch gekennzeichnet, daß** die Einrichtung (17) zur Überwachung des Gefäßzuganges Mittel (23) zum Speichern der in aufeinanderfolgenden Messungen ermittelten Summen und Differenzen des venösen und arteriellen Drucks aufweist und daß die Recheneinheit (22) zur Berechnung eines ersten den Zustand des Gefäßzuganges charakteristischen Wertes $W_1$ Mittel zum Bilden der Differenz zwischen der Summe des venösen und arteriellen Drucks einer nachfolgenden Messung und der Summe des venösen und arteriellen Drucks einer vorhergehenden Messung und Mittel zum Addieren dieser Druckdifferenzen aufeinanderfolgender Messungen aufweist und zur Berechnung eines zweiten den Zustand des Gefäßzuganges charakteristischen Wertes $W_2$ Mittel zum Bilden der Differenz zwischen der Differenz des venösen und arteriellen Drucks einer nachfolgenden Messung und der Differenz des venösen und arteriellen Drucks einer vorhergehenden Messung und Mittel zum Addieren dieser Druckdifferenzen aufeinanderfolgender Messungen aufweist.

3. Vorrichtung nach Anspruch 2 **dadurch gekennzeichnet, daß** die Auswerteinheit (25) Mittel zum Bestimmen des Vorzeichens des ersten und zweiten charakteristischen Wertes $W_1$, $W_2$ und Mittel zum Vergleichen des ersten charakteristischen Wertes mit einem ersten Schwellwert $M_1$ und Mittel zum Vergleichen des zweiten charakteristischen Wertes $W_2$ mit einem zweiten Schwellwert $M_2$ aufweist.

4. Vorrichtung nach einem der Ansprüche 1 bis 3, **dadurch gekennzeichnet, daß** ein Alarmgeber (28) vorgesehen ist, der bei Feststellung eines fehlerhaften Gefäßzuganges einen Alarm auslöst.

5. Vorrichtung nach einem der Ansprüche 1 bis 4, **dadurch gekennzeichnet, daß** Mittel (16, 28) zum Unterbrechen des Blutflusses im extrakorporalen Blutkreislauf bei der Feststellung eines fehlerhaften Gefäßzuganges vorgesehen sind.

**Claims**

1. A device for extracorporeal blood treatment with an arterial blood line (5) of an extracorporeal blood circuit, which is connected at one end to the inlet of a blood treatment apparatus (1) and is provided at the other end with an arterial connection (5a) for a vessel access,
a venous blood line (7) of an extracorporeal blood circuit, which is connected at one end to the outlet of a blood treatment apparatus (1) and is provided at the other end with a venous connection (7a) for the vessel access, and
a device (17) for monitoring the vessel access with an arterial pressure sensor (18) monitoring pressure $P_{art.}$ in the arterial blood line and a venous pressure sensor (19) monitoring pressure $P_{ven.}$ in the venous blood line,
**characterised in that**
the device for monitoring the vessel access has:

a computing unit (22) with means for calculating the sum and
the difference of the venous and arterial pressure in the extracorporeal circuit detected in successive measurements and means for calculating values $W_1$, $W_2$, characteristic of the state of the vessel access, from the determined sums and

differences of the detected pressure values in the arterial and venous branch of the extracorporeal circuit and an analysing unit (25) for analysing the characteristic values in order to identify a defective vessel access.

2. The device according to claim 1, **characterised in that** the device (17) for monitoring the vessel access has means (23) for storing the sums and differences of the venous and arterial pressure determined in successive measurements and that the computing unit (22) has means for finding the difference between the sum of the venous and arterial pressure of a subsequent measurement and the sum of the venous and arterial pressure of a preceding measurement and means for adding up these pressure differences of successive measurements for the purpose of calculating a first value $W_1$ characteristic of the state of the vessel access and means for finding the difference between the difference of the venous and arterial pressure of a subsequent measurement and the difference of the venous and arterial pressure of a preceding measurement and means for adding up these pressure differences of successive measurements for the purpose of calculating a second value $W_2$ characteristic of the state of the vessel access.

3. The device according to claim 2, **characterised in that** the analysing unit (25) has means for determining the sign of the first and second characteristic values $W_1$, $W_2$ and means for comparing the first characteristic value with a first threshold value $M_1$ and means for comparing second characteristic value $W_2$ with a second threshold value $M_2$.

4. The device according to any one of claims 1 to 3, **characterised in that** an alarm transmitter (28) is provided, which triggers an alarm when a defective vessel access is ascertained.

5. The device according to any one of claims 1 to 4, **characterised in that** means (16, 28) are provided for interrupting the blood flow in the extracorporeal blood circuit when a defective vessel access is ascertained.

**Revendications**

1. Dispositif de traitement sanguin extracorporel avec une conduite de sang artérielle (5) d'un circuit sanguin extracorporel, qui est reliée, à une extrémité, à l'entrée d'un dispositif de traitement sanguin (1) et qui est dotée à l'autre extrémité d'un raccord artériel (5a) pour un accès à un vaisseau sanguin,

une conduite de sang veineuse (7) du circuit sanguin extracorporel, qui est reliée, à une extrémité, à la sortie du dispositif de traitement sanguin (1) et qui est dotée à l'autre extrémité d'un raccord veineux (7a) pour l'accès à un vaisseau sanguin, et

une installation (17) de contrôle de l'accès à un vaisseau sanguin avec un capteur de pression artérielle (18) contrôlant la pression $P_{art.}$ dans la conduite sanguine artérielle, et un capteur de pression veineuse (19) contrôlant la pression $P_{vein.}$ dans la conduite sanguine veineuse,

   **caractérisé en ce que**,

l'installation de contrôle de l'accès à un vaisseau sanguin comprend :

une unité de calcul (22) avec un moyen d'évaluation de la somme et de la différence des pressions veineuse et artérielle détectées dans les mesures successives dans le circuit extracorporel et des moyens d'évaluation des valeurs $W_1$, $W_2$ caractéristiques de l'état d'accès à un vaisseau sanguin à partir des sommes et différences déterminées des valeurs de pression détectées dans le bras veineux et artériel du circuit extracorporel et

une unité d'évaluation (25) pour l'évaluation des valeurs caractéristiques afin de déceler un accès défectueux à un vaisseau sanguin.

2. Dispositif selon la revendication 1, **caractérisé en ce que** l'installation (17) de contrôle de l'accès à un vaisseau sanguin comprend un moyen (23) d'enregistrement des sommes et différences déterminées dans les mesures successives de la pression veineuse et artérielle, et **en ce que** l'unité de calcul (22) pour le calcul d'une première valeur caractéristiques $W_1$ de l'état de l'accès à un vaisseau sanguin comprend un moyen de formation de la différence entre la somme de la pression veineuse et artérielle d'une mesure suivante et la somme de la pression veineuse et artérielle d'une mesure précédente, et un moyen d'ajout de ces différences de pression des mesures successives, et pour le calcul d'une seconde valeur $W_2$ caractéristique de l'état de l'accès à un vaisseau sanguin pour la formation de la différence entre la différence de la pression veineuse et artérielle d'une mesure suivante et la différence de la pression veineuse et artérielle d'une mesure précédente, et un moyen d'ajout de ces différences de pression des mesures successives.

3. Dispositif selon la revendication 2, **caractérisé en ce que** l'unité d'évaluation (25) comprend un moyen de détermination du traçage de la première et de la seconde valeur caractéristiques $W_1$, $W_2$ et un

moyen de comparaison de la première valeur caractéristique à une première valeur de seuil $M_1$ et un moyen de comparaison de la seconde valeur caractéristique $W_2$ à une seconde valeur de seuil $M_2$.

4. Dispositif selon l'une des revendications 1 à 3, **caractérisé en ce qu'**il est prévu un dispositif d'alarme (28) qui déclenche une alarme lorsqu'un accès défectueux à un vaisseau sanguin est constaté.

5. Dispositif selon l'une des revendications 1 à 4, **caractérisé en ce que** des moyens (16, 28) d'interruption de la circulation sanguine dans le circuit sanguin extracorporel sont prévus lorsqu'un accès défectueux à un vaisseau sanguin est constaté.

| Ursache | $P_{art.}$ | $P_{ven.}$ | $P_s$ | $\Delta P$ | $\sum_N d(P_s)$ | $\sum_N d(\Delta P)$ | Bemerkung |
|---|---|---|---|---|---|---|---|
| 1. Blutdruckabfall des Patienten | ↓ | ↓ | ↓ | → | · | 0 | |
| 2. Blutdruckanstieg des Patienten | ↑ | ↑ | ↑ | → | + | 0 | |
| 3. Viskositätserhöhung des Blutes | ↓ | ↑ | ↓ | ↑ | · | + | |
| 4. Viskositätserniedrigung des Blutes | ↑ | ↓ | ↑ | ↓ | + | · | |
| 5. Erhöhung von $Q_s$ | ↓ | ↑ | ↓ | ↑ | · | + | Abb. 4 |
| 6. Erniedrigung von $Q_s$ | ↑ | ↓ | ↑ | ↓ | + | · | Abb. 4 |
| 7. Lageänderung des Patienten nach oben | ↑ | ↑ | ↑ | → | + | 0 | |
| 8. Lageänderung des Patienten nach unten | ↓ | ↓ | ↓ | → | · | 0 | Abb. 5 |
| 9. Blutleck im arteriellen Schlauchsystem | ↑ | → | ↑ | ↑ | + | + | Durch vorhandene Schutzsysteme gesichert |
| 10. Blutleck im venösen Schlauchsystem | → | ↓ | ↓ | ↓ | · | · | Abb. 6; Unzureichend gesichert! |
| 11. Herausrutschen der arteriellen Kanüle | ↑ | → | ↑ | ↑ | + | + | Durch vorhandene Schutzsysteme gesichert |
| 12. Herausrutschen der venösen Kanüle | ↓ | ↓ | ↓ | ↓ | · | · | Abb. 6; Unzureichend gesichert! |

Einflüsse, die zur Änderung der Drucke im extrakorporalen Kreislauf beitragen.

↑ : Anstieg

↓ : Abfall

→ : Keine Änderung

Figur 1

EP 0 995 451 B1

Figur 2

EP 0 995 451 B1

Figur 3

Figur 4

Figur 5

13

EP 0 995 451 B1

Herauszlehen der venösen Kanüle

Figur 6

Blutleck im venösen Schlauchsystem    Verschluß des Lecks

Figur 7

14